# EUROPEAN PATENT APPLICATION

(11) **EP 1 291 354 A2**
(43) Date of publication of application: **12.03.2003**
(21) Application number: 02079758.5
(22) Date of filing: 10.03.2000
(51) Int. Cl.: C07H 19/00, C12Q 1/68

(54) **Nucleotide compounds having a cleavable linker**

(30) Priority: 12.03.1999 US 267496
(62) Divisional of application: 00917853.4
(71) Applicant: PRESIDENT AND FELLOWS OF HARVARD COLLEGE, Cambridge Massachusetts 02138 (US)
(72) Inventor: Church, George M., Brookline, MA 02146 (US); Mitra, Robi D., Chestnut Hill, MA 02147 (US)
(74) Representative: Prins, Adrianus Willem

(57) **Abstract**

It has turned out to be valuable to use a disulfide-bonded cleavable nucleotide complex according formula I

Dye - SS - Linker - Nucleotide (formula I)
wherein
Dye is a fluorophore;
SS is a disulfide bridge;
Linker is a useful linking group, such as - C ≡ C - CH₂ - NH - CO - CH₂ - ; and Nucleotide is a nucleotide chosen from the group of dCTP, dATP, dGTP, dTTP, ribonucleotides or further modified nucleotides;
in nucleic acid assay methods.

SEQUENCE LISTING

<110> Church, George

Mitra, Robi

<120> Nucleotide Compounds Having A Cleavable Linker

<130> 10498-00043

<140> TBA

<141> herewith

<160> 3

<170> PatentIn version 3.1

<210> 1

<211> 23

<212> DNA

<213> Artificial Sequence

<220>

<223> amplification primer

<400> 1
gcccggtctc gagcgtctgt tta 23

<210> 2

<211> 60

<212> DNA

<213> Artificial Sequence

<220>

<223>  amplification primer

<220>

<221>  modified_base

<222>  (1)..(1)

<223>

<400>  2
tcggccaacg cgcggggaga ggcggtttgc gtatcagtaa acagacgctc gagaccgggc      60

<210>  3

<211>  60

<212>  DNA

<213>  Artificial Sequence

<220>

<223>  amplification primer

<220>

<221>  modified_base

<222>  (1)..(1)

<223>

<400>  3
cccagtcacg acgttgtaaa acgacggcca gtgtcgataa acagacgctc gagaccgggc      60

## Description

### FIELD OF THE INVENTION

The invention relates in general to nucleotides joined to a fluorophore by a cleavable linkage. More specifically, the cleavable linkage is a disulfide linkage. The invention also relates to methods of sequencing nucleic acids on arrays.

### BACKGROUND OF THE INVENTION

Oligonucleotide probes may be labeled in a variety of ways, including the direct or indirect attachment of radioactive moieties, fluorescent moieties, colorimetric moieties, and the like. Many comprehensive reviews of methodologies for labeling DNA and constructing DNA probes provide guidance applicable to constructing probes (see Matthews et al., 1988, Anal. Biochem., 169: 1-25; Haugland, 1992, Handbook of Fluorescent Probes and Research Chemicals, Molecular Press, Inc., Eugene, OR; Keller and Manak, 1993, DNA Probes, 2nd Ed., Stockton Press, New York; Eckstein, ed., 1991, Oligonucleotides and Analogues: A Practical Approach, ML Press, Oxford, 1991); Wetmur, 1991, Critical Reviews in Biochemistry and Molecular Biology, 26: 227-259). Many more particular labeling methodologies are known in the art (see Connolly, 1987, Nucleic Acids Res., 15: 3131-3139; Gibson et al. 1987, Nucleic Acids Res., 15: 5455-6467; Spoat et al., 1987, Nucleic Acids Res., 15: 4837-4848; Fung et al., U.S. Pat. No. 4,757,141; Hobbs, et al., U.S. Pat No. 5,151,507; Cruickshank, U.S. Pat. No. 5,091,519 (synthesis of functionalized oligonucleotides for attachment of reporter groups); Jablonski et al., 1986, Nucleic Acids Res., 14: 6115-6128 (enzyme/oligonucleotide conjugates); and Urdea et al., U.S. Pat. No. 5,124,246 (branched DNA)).

There is also a need in the art for improved methods of sequencing the molecules on nucleic acid arrays.

### Summary of the Invention

It has turned out to be valuable to use a disulfide-bonded cleavable nucleotide complex according to formula I
Dye ― SS ― Linker ― Nucleotide (formula I)
wherein
Dye is a fluorophore;
SS is a disulfide bridge;
Linker is a useful linking group, such as - C ≡ C ― CH₂ ― NH ― CO -― CH₂ ― ; and
Nucleotide is a nucleotide chosen from the group of dCTP, dATP, dGTP, dTTP, ribonucleotides or further modified nucleotides;
in nucleic acid assay methods. The fluorophore is chosen from the group of cyanine 5 or cyanine 3, in a preferred method the fluorophore is obtainable from New England Nuclear ― DuPont.

Furthermore, it has turned out that there is a need for improved nucleotide sequencing methods that don't require electrophoretic separation of closely-sized DNA fragments, since it is difficult to automate gel-based separations, or the generation of nested deletions of the target polynucleotide. In addition, detection and analysis are greatly simplified because signal-to noise ratios are much more favorable on a nucleotide-by-nucleotide basis, permitting smaller sample sizes to be employed. For fluorescent-based detection schemes, analysis is further simplified because fluorophores labeling different nucleotides may be separately detected in homogeneous solutions rather than in spatially overlapping bands.

### Detailed Description of the Invention

Cleavable linkages between the fluorophore and the nucleotide may be employed to permit removal of the fluorophore after incorporation and detection, thereby setting the sequence up for additional labeled base addition and detection. As used herein, the term "cleavable linkage" refers to a chemical moiety that joins a fluorophore to a nucleotide, and that can be cleaved to remove the fluorophore from the nucleotide when desired, essentially without altering the nucleotide or the nucleic acid molecule it is attached to. Cleavage may be accomplished, for example, by acid or base treatment, or by oxidation or reduction of the linkage, or by light treatment (photobleaching), depending upon the nature of the linkage. Examples of cleavable linkages are described by Shimkus et al., 1985, Proc. Natl. Acad. Sci. USA 82: 2593-2597; Soukup et al., 1995, Bioconjug. Chem. 6: 135-138; Shimkus et al., 1986, DNA 5: 247-255; and Herman and Fenn, 1990, Meth. Enzvmol. 184: 584-588, all of which are incorporated herein by reference.

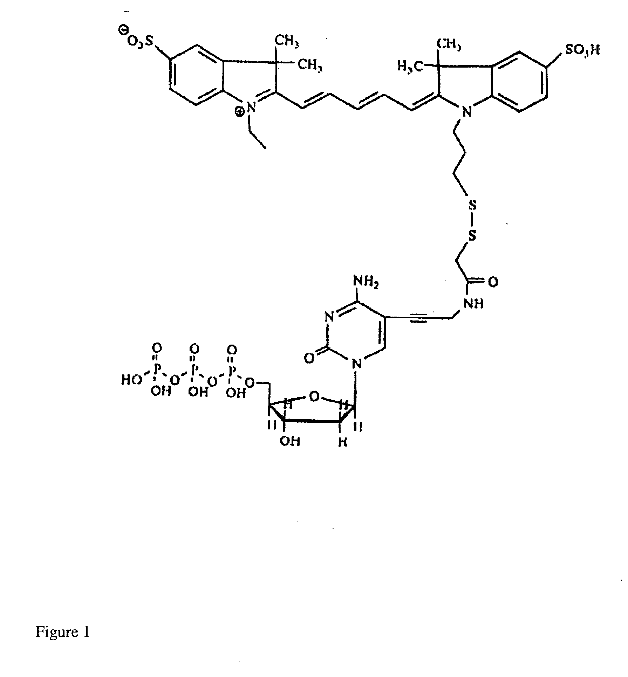

According to the present invention, a cleavable disulfide linkage may be reduced using thiol compound reducing agents such as dithiothreitol. Fluorophores are available with a sulfhydryl (SH) group available for conjugation (e.g., Cyanine 5 or Cyanine 3 fluorophores with SH groups; New England Nuclear ― DuPont), as are nucleotides with a reactive aryl amino group (e.g., dCTP). A reactive pyridyldithiol will react with a sulfhydryl group to give a sulfhydryl bond that is cleavable with reducing agents such as dithiothreitol. An NHS-ester heterobifunctional crosslinker (Pierce) is used to link a deoxynucleotide comprising a reactive aryl amino group to a pyridyldithiol group, which is in turn reactive with the SH on a fluorophore, to yield a disulfide bonded, cleavable nucleotide-fluorophore complex useful in the methods of the invention (see, for example, Figure 1).

Accordingly, the present invention provides nucleotides joined to a fluorophore by a cleavable disulfide linkage. Such nucleotides can advantageously be used in a method of determining the nucleotide sequence of the features of an array of immobilized nucleic acids comprising the steps of: a) adding a mixture comprising an oligonucleotide primer and a template-dependent polymerase to an array of immobilized nucleic acid features under conditions permitting hybridization of the primer to the immobilized nucleic acids; b) adding a single, fluorescently labeled deoxynucleoside triphosphate to the mixture under conditions which permit incorporation of the labeled deoxynucleotide onto the 3' end of the primer if it is complementary to the next adjacent base in the sequence to be determined; c) detecting incorporated label by monitoring fluorescence; d) repeating steps (b) - (c) with each of the remaining three labeled deoxynucleoside triphosphates in turn; and e) repeating steps (b) - (d) until the nucleotide sequence is determined.

Alternatively, the primer, buffer and polymerase are cast into a polyacrylamide gel bearing the array of immobilized nucleic acids.

It is preferred that the single fluorescently labeled deoxynucleotide further comprises a mixture of the single deoxynucleoside triphosphate in labeled and unlabeled forms.

As already mentioned, the fluorescently labeled deoxynucleoside triphosphates are labeled with a cleavable linkage to the fluorophore, and the additional step of cleaving said linkage to the fluorophore is performed after step (d) and before step (e).

The nucleotides of the invention can also be used in a method of determining the nucleotide sequence of the features of an array of immobilized nucleic acids comprising the steps of: a) adding a mixture comprising an oligonucleotide primer and a template-dependent polymerase to an array of immobilized nucleic acids; b) adding a first mixture of three unlabeled deoxynucleoside triphosphates under conditions which permit incorporation of deoxynucleotides to the end of the primer if the are complementary to the next adjacent base in the sequence to be determined; c) adding a second mixture of three unlabeled deoxynucleoside triphosphates, along with buffer and polymerase if necessary, said second mixture comprising the deoxynucleoside triphosphate not included in the mixture of step (b), under conditions which permit incorporation of deoxynucleotides to the end of the primer if they are complementary to the next adjacent base in the sequence to be determined; d) repeating steps (b) - (c) for a predetermined number of cycles; e) adding a single, fluorescently labeled deoxynucleoside triphosphate of the invention to the mixture under conditions which permit incorporation of the labeled deoxynucleotide onto the 3' terminus of the primer if it is complementary to the next adjacent base in the sequence to be determined; f) detecting incorporated label by monitoring fluorescence; g) repeating steps (e) - (f), with each of the remaining three labeled deoxynucleoside triphosphates in turn; and h) repeating steps (e) - (g) until the nucleotide sequence is determined.

It is preferred that the first or second mixture of three unlabeled deoxynucleoside triphosphates, a mixture, which comprises deoxyguanosine triphosphate, further comprises de-oxyadenosine triphosphate.

In a preferred embodiment, method the primer and polymerase are cast into a polyacrylamide gel bearing the array of immobilized nucleic acids.

In a preferred embodiment, the single fluorescently labeled deoxynucleotide further comprises a mixture of the single deoxynucleoside triphosphate in labeled and unlabeled forms.

In all embodiment of this method of determining the nucleotide sequence of nucleic acid features on an array, the fluorescently labeled deoxynucleoside triphosphates are labeled with a cleavable linkage to the fluorophore and after step (g) and before step (h) the step of cleaving the linkage to the fluorophore is performed.

Furthermore, in all embodiment of this method detection prior to cleavage is preferred where sequencing is carried out in parallel on a plurality of sequences (either segments of a single target polynucleotide or a plurality of altogether different target polynucleotides), e.g. attached to separate magnetic beads, or other types of solid phase supports.

As alluded to, the target polynucleotide may be anchored to a solid-phase support, such as a magnetic particle, polymeric microsphere, filter material, or the like, which permits the sequential application of reagents without complicated and time-consuming purification steps. The length of the target polynucleotide can vary widely; however, for convenience of preparation, lengths employed in conventional sequencing are preferred. For example, lengths in the range of a few hundred basepairs, 200-300, to 1 to 2 kilobase pairs are most often used.

As used herein in reference to nucleic acid arrays, the term "plurality" is defined as designating two or more such arrays, wherein a first (or "template") array plus a second array made from it comprise a plurality. When such a plurality comprises more than two arrays, arrays beyond the second array may be produced using either the first array or any copy of it as a template.

As used herein, the terms "randomly-patterned" or "random" refer to a non-ordered, non-Cartesian distribution (in other words, not arranged at pre-determined points along the x-and y axes of a grid or at defined "clock positions", degrees or radii from the center of a radial pattern) of nucleic acid molecules over a support, that is not achieved through an intentional design (or program by which such a design may be achieved) or by placement of individual nucleic acid features. Such a "randomly-patterned" or "random" array of nucleic acids may be achieved by dropping, spraying, plating or spreading a solution, emulsion, aerosol, vapor or dry preparation comprising a pool of nucleic acid molecules onto a support and allowing the nucleic acid molecules to settle onto the support without intervention in any manner to direct them to specific sites thereon.

As used herein, the terms "immobilized" or "affixed" refer to covalent linkage between a nucleic acid molecule and a support matrix.

As used herein, the term "array" refers to a heterogeneous pool of nucleic acid molecules that is distributed over a support matrix; preferably, these molecules differing in sequence are spaced at a distance from one another sufficient to permit the identification of discrete features of the array.

As used herein, the term "heterogeneous" is defined to refer to a population or collection of nucleic acid molecules that comprises a plurality of different sequences; it is contemplated that a heterogeneous pool of nucleic acid molecules results from a preparation of RNA or DNA from a cell which may be unfractionated or partially-fractionated.

An "unfractionated" nucleic acid preparation is defined as that which has not undergone the selective removal of any sequences present in the complement of RNA or DNA, as the case may be, of the biological sample from which it was prepared. A nucleic acid preparation in which the average molecular weight has been lowered by cleaving the component nucleic acid molecules, but which still retains all sequences, is still "unfractionated" according to this definition, as it retains the diversity of sequences present in the biological sample from which it was prepared.

A "partially-fractionated" nucleic acid preparation may have undergone qualitative size-selection. In this case, uncleaved sequences, such as whole chromosomes or RNA molecules, are selectively retained or removed based upon size. In addition, a "partially-fractionated" preparation may comprise molecules that have undergone selection through hybridization to a sequence of interest; alternatively, a "partially-fractionated" preparation may have had undesirable sequences removed through hybridization. It is contemplated that a "partially-fractionated" pool of nucleic acid molecules will not comprise a single sequence that has been enriched after extraction from the biological sample to the point at which it is pure, or substantially pure.

In this context, "substantially pure" refers to a single nucleic acid sequence that is represented by a majority of nucleic acid molecules of the pool. Again, this refers to enrichment of a sequence *in vitro*; obviously, if a given sequence is heavily represented in the biological sample, a preparation containing it is not excluded from use according to the invention.

As used herein, the term "biological sample" refers to a whole organism or a subset of its tissues, cells or component parts (e.g. fluids). "Biological sample" further refers to a homogenate, lysate or extract prepared from a whole organism or a subset of its tissues, cells or component parts, or a fraction or portion thereof. Lastly, "biological sample" refers to a medium, such as a nutrient broth or gel in which an organism has been propagated, which contains cellular components, such as nucleic acid molecules.

As used herein, the term "organism" refers to all cellular life-forms, such as prokaryotes and eukaryotes, as well as non-cellular, nucleic acid-containing entities, such as bacteriophage and viruses.

As used herein, the term "feature" refers to each nucleic acid sequence occupying a discrete physical lotion on the array; if a given sequence is represented at more than one such site, each site is classified as a feature. In this context, the term "nucleic acid sequence" may refer either to a single nucleic acid molecule, whether double or single-stranded, to a "clone" of amplified copies of a nucleic acid molecule present at the same physical location on the array or to a replica, on a separate support, of such a clone.

As used herein, the term "amplifying" refers to production of copies of a nucleic acid molecule of the array *via* repeated sounds of primed enzymatic synthesis; *"in situ* amplification" indicates that such amplifying takes place with the template nucleic acid molecule positioned on a support according to the invention, rather than in solution.

As used herein, the term "support" refers to a matrix upon which nucleic acid molecules of a nucleic acid array are immobilized; preferably, a support is semi-solid.

As used herein, the term "semi-solid" refers to a compressible matrix with both a solid and a liquid component, wherein the liquid occupies pores, spaces or other interstices between the solid matrix elements.

As used herein in reference to the physical placement of nucleic acid molecules or features and/or their orientation relative to one another on an array of the invention, the terms "correspond" or "corresponding" refer to a molecule occupying a position on a second array that is either identical to- or a mirror image of the position of a molecule from which it was amplified on a first array which served as a template for the production of the second array, or vice versa, such that the arrangement of features of the array relative to one another is conserved between arrays of a plurality.

As implied by the above statement, a first and second array of a plurality of nucleic acid arrays according to the invention may be of either like or opposite chirality, that is, the patterning of the nucleic acid arrays may be either identical or mirror-imaged.

As used herein, the term "replica" refers to any nucleic acid array that is produced by a printing process according to the invention using as a template a first randomly-patterned immobilized nucleic acid array.

As used herein, the term "spot" as applied to a component of a micro-array refers to a discrete area of a surface containing a substance deposited by mechanical or other means.

As used herein, the term "excluded volume" refers to the volume of space occupied by a particular molecule to the exclusion of other such molecules.

As used herein, "excess of nucleic acid molecules" refers to an amount of nucleic acid molecules greater than the amount of entities to which such nucleic acid molecules may bind. An excess may comprise as few as one molecule more than the number of binding entities, to twice the number of binding entities, up to 10 times, 100 times, 1000 times the number of binding entities or more.

As used herein, "signal amplification method" refers to any method by which the detection of a nucleic acid is accomplished.

As used herein, a "nucleic acid capture ligand" or "nucleic acid capture activity" refers to any substance which binds nucleic acid molecules, either specifically or non-specifically, or which binds an affinity tag attached to a nucleic acid molecule in such a way as to immobilize the nucleic acid molecule to a support bearing the capture ligand.

As used herein, "replica-destructive" refers to methods of signal amplification, which render an array or replica of an array non-reusable.

As used herein, the term "non-reusable", in reference to an array or replica of an array, indicates that, due to the nature of detection methods employed, the array cannot be replicated nor used for subsequent detection methods after the first detection method is performed.

As used herein, the term "essentially distinct" as applied to features of an array refers to the situation where 90% or more of the features of an array are not in contact with other features on the same array.

As used herein, the term "preserved" as applied to the resolution of nucleic acid features on an array means that the features remain essentially distinct after a given process has been performed.

As used herein, the term "distinguishable" as applied to a label, refers to a labeling moiety, which can be detected when among other labeling moieties.

As used herein, the term "spectrally distinguishable" or "spectrally resolvable" as applied to a label, refers to a labeling moiety which can be detected by its characteristic fluorescent excitation or emission spectra, one or both of such spectra distinguishing said moiety from other moieties used separately or simultaneously in the particular method.

As used herein, the term "chain-terminating analog" refers to any nucleotide analog, which once incorporated onto the 3' end of a nucleic acid molecule, cannot serve as a substrate for further addition of nucleotides to that nucleic acid molecule.

It is preferred that the support is semi-solid.

Preferably, the semi-solid support is selected from the group that includes polyacrylamide, cellulose, polyamide (nylon) and cross-linked agarose, -dextran and -polyethylene glycol.

It is particularly preferred that amplifying of nucleic acid molecules are performed by polymerase chain reaction (PCR).

Preferably, affixing of nucleic acid molecules to the support is performed using a covalent linker that is selected from the group that includes oxidized 3-methyl uridine, an acrylyl group and hexaethylene glycol. Additionally, ACRYDITE oligonucleotide primers may be covalently fixed within a polyacrylamide gel.

It is also contemplated that affixing of nucleic acid molecules to the support is performed *via* hybridization of the members of the pool to nucleic acid molecules that are covalently bound to the support.

As used herein, the term "synthetic oligonucleotide" refers to a short (10 to 1,000 nucleotides in length), double- or single-stranded nucleic acid molecule that is chemically synthesized or is the product of a biological system such as a product of primed or unprimed enzymatic synthesis.

As used herein, the term "template DNA" refers to a plurality of DNA molecules used as the starting material or template for manufacture of a nucleic acid array such as a poly-acrylamide-immobilized nucleic acid array.

As used herein, the term "template nucleic acids" refers to plurality of nucleic acid molecules used as the starting material or template for manufacture of a nucleic acid array.

As used herein, the term "amplification primer" refers to an oligonucleotide that may be used as a primer for amplification reactions. The term "PCR primer" refers to an oligonucleotide that may be used as a primer for the polymerase chain reaction. A PCR primer is preferably, but not necessarily, synthetic, and will generally be approximately 10 to 100 nucleotides in length.

As used herein, the term "ACRYDITE modified" in reference to an oligonucleotide means that the oligonucleotide has an ACRYDITE phosphoramidite group attached to the 5' end of the molecule.

As used herein, the term "thermostable, template-dependent DNA polymerase" refers to an enzyme capable of conducting primed enzymatic synthesis following incubation at a temperature, greater than 65°C and less than or equal to approximately 100°C, and for a time, ranging from about 15 seconds to about 5 minutes, that is sufficient to denature essentially all double stranded DNA molecules in a given population.

As used herein, the term "solid support" refers to a support for a polyacrylamide-immobilized nucleic acid array, such support being essentially non-compressible and lacking pores containing liquid. A solid support is preferably thin and thermally conductive, such that changes in thermal energy characteristic of PCR thermal cycling are conducted through the support to permit amplification of PCR template molecules arrayed on its surface.

As used herein, the term "binding sites" when used in reference to a nucleic acid molecule, means sequences that hybridize under selected PCR annealing conditions with a selected primer. Binding sites for PCR primers are generally used in pairs situated on either side of a sequence to be amplified, with each member of the pair preferably comprising a sequence from the other member of the pair.

As used herein, the term "variable sequence" refers to a sequence in a population of nucleic acid molecules that varies between different members of the population. Generally, as used herein, a variable sequence is flanked on either side by sequences that are shared or constant among all members of that population.

### BRIEF DESCRIPTION OF THE DRAWINGS

Figure 1 is a schematic drawing of a disulfide-bonded cleavable nucleotide fluorophore complex useful in the methods of the invention.

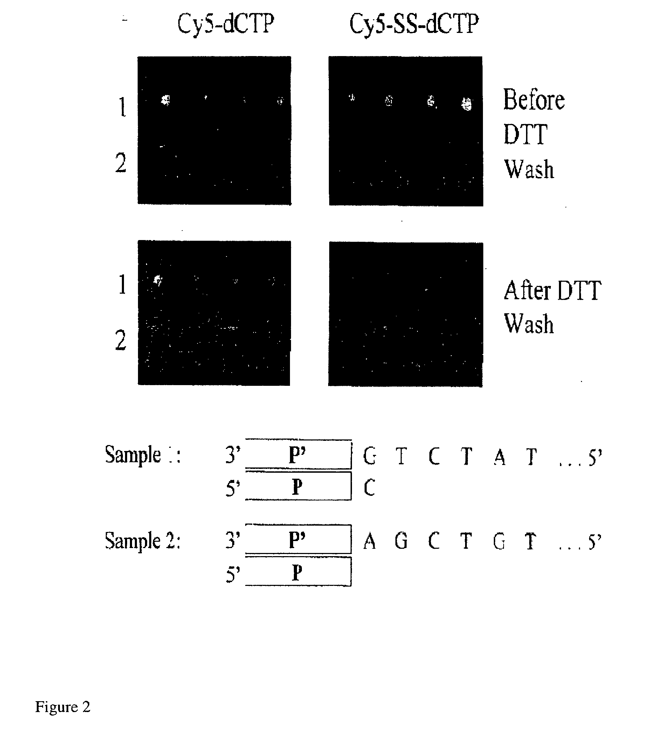

Figure 2 shows the results of experiments establishing the function of cleavable linkers in polyacrylamide gel matrix fluorescent sequencing reactions. The top panels show fluorescent scans of primer extension reactions, on two separate sequencing templates, in polyacrylamide spots using nucleotides with non-cleavable (Cy5-dCTP) and cleavable (Cy5-SS-dCTP) linked fluorescent label, before and after cleavage with dithiothreitol (DTT). The bottom panel shows schematics of sequencing templates 1 and 2 with the expected extension products.

### EXAMPLE 1

### Fluorescent in Situ Sequencing Extension Quantification with Cleavable Linkers

The method of sequencing nucleic acid molecules within a polyacrylamide gel matrix using the Fluorescent In Situ Sequencing Extension Quantification method and nucleotides labeled with cleavable linkers was demonstrated in the following experiments.

In order to evaluate the method, molecules of a known DNA sequence were first cast into a polyacrylamide gel matrix. The oligonucleotide sequencing primer RMGP1-R (5'-gcc cgg tct cga gcg tct gtt ta (SEQ ID NO:1)) was annealed to the oligonucleotide puc514c (Q - 5' tcggcc aacgcggg gagaggeggt ttgcgtatca g **taaacagac gctcgagacc gggc** (SEQ ID NO:2) (sample 1)) or the oligonucleotide puc234t (Q - 5' ccagt cacgacgttg taaaacgacg gccagtgtcg a **taaacagac gctcgagacc gggc** (SEQ ID NO:3) (sample 2). The bolded sequences denote the sequences to which the sequencing primer anneals, and Q indicates an ACRYDITE modification.

Equal amounts of template and primer were annealed at a final concentration of 5 µM in 1x EcoPol buffer (10 mM Tris pH 7.5, 5 mM MgCl₂), by heating to 95 degrees C for 1 minute, slowly cooling to 50 degrees C at a rate of 0.1 degrees per second, and holding the reaction at 50 degrees C for 5 minutes. The primer:template complex was then diluted by adding 30 µl 1x EcoPol buffer and 2 µl 500 mM EDTA.

One microliter of each annealed oligonucleotide was added to 17 µl of acrylamide gel mixture (40 mM Tris pH 7.3, 25% glycerol, 1 mM DTT, 6% acrylamide (5% cross-linking), 17.4 units SEQUENASE version 2.0 (United States Biochemical, USB), 15 µg/ml E. *coli* single stranded binding protein (USB), 0,1 mg/ml BSA). Then, 1 µl of 1.66% TEMED and 1 µl of 1.66% APS were added and 0.2 µl of each mixture was pipetted onto bind-silane treated glass microscope slides. The slides were immediately put under an argon bed for 30 minutes to allow polymerization of the acrylamide.

The slides containing the spots of polyacrylamide containing DNA molecules to be sequenced were then washed in 40 mM Tris pH 7.5, 0,01% Triton X-100 for 30 seconds, after which they were ready for the incorporation of labeled nucleotides. For this experiment, dCTP labeled with the fluorophore Cy5 with either a non-cleavable linkage (referred to herein as Cy5-dCTP) or with a disulfide-containing cleavable linkage (referred to herein as Cy5-SS-dCTP) was used. The acrylamide spots containing known DNA to be sequenced were incubated in 30 µl of Cy-5 dCTP extension mix (10 mM Tris pH 7.5, 50 mM NaCl, 5 mM MgCl₂, 0,1 mg/ml BSA, 0,01% Triton X-100, 0.1 µM unlabeled dCTP,0.2 µM Cy5-dCTP) or in Cy-5-SS-dCTP extension mix (10 mM Tris pH 7.5, 50 mM NaCI, 5 mM MgCl₂, 0,1 mg/ml BSA, 0,01% Triton X-100, 0.1 µM unlabeled dCTP,0.2 µM Cy5-SS-dCTP) for 4 minutes at room temperature. The slides were washed twice, for 5 minutes each in FISSEQ wash buffer (10 mM Tris pH 7.5, 250 mM NaCl, 2 mM EDTA, 0,01% Triton X-100), spun briefly to dry and scanned on a Scanarray 4000 confocal scanner (GSI Luminomics). The settings were as follows: Focus = 260, Laser = 80%, PMT = 80% resolution = 30 microns.

Cleavage of the cleavable disulfide linkages was performed by incubation with the reducing agent dithiothreitol (DTT). The slides were incubated overnight in FISSEQ wash buffer supplemented with 5 mM DTT, washed twice for 5 minutes each in wash buffer, spun briefly to dry and scanned as before. Figure 2 shows the results of this experiment. Sample 1 incorporated both the cleavable and the non-cleavable fluorescently labeled nucleotide (see "Before DTT Wash" panels"), while sample 2 did not, as was expected since only sample 1 had a G as the next template nucleotide. DTT wash (bottom panels) removed the fluorescent signal from the samples extended with the Cy5-SS-dCTP sample, but not from the samples extended with the non-cleavable linked fluorophore, demonstrating that the cleavable linkages could be cleaved, or chemically bleached, from the Cy5-SS-dCTP-extended samples with reducing agent, but not from the Cy5-dCTP-extended samples. One of skill in the art would fully expect similar cleavable linkages to nucleotides other than dCTP (for example, dATP, dGTP, TTP or even ribonucleotides or further modified nucleotides) to function in a similar manner.

## Claims

**1.** A disulfide-bonded cleavable nucleotide complex according to formula I
Dye ― SS ― Linker ― Nucleotide (formula I)
wherein
- Dye is a fluorophore;
- SS is a disulfide bridge;
- Linker is a useful linking group, such as - C ≡ C ― CH₂ ― NH ― CO ― CH₂―; - Nucleotide is a nucleotide chosen from the group of dCTP, dATP, dGTP, dTTP, ribonucleotides or further modified nucleotides.

**2.** A nucleotide complex according to claim 1, **characterised in that** the fluorophore is chosen from the group of cyanine 5 or cyanine 3.

**3.** A nucleotide complex according to claim 1, **characterised in that** the fluorophore is chosen from the group of cyanine 5 or cyanine 3 obtainable from New England Nuclear - DuPont.

**4.** A nucleotide complex according to claim 1, having the structure of formula II 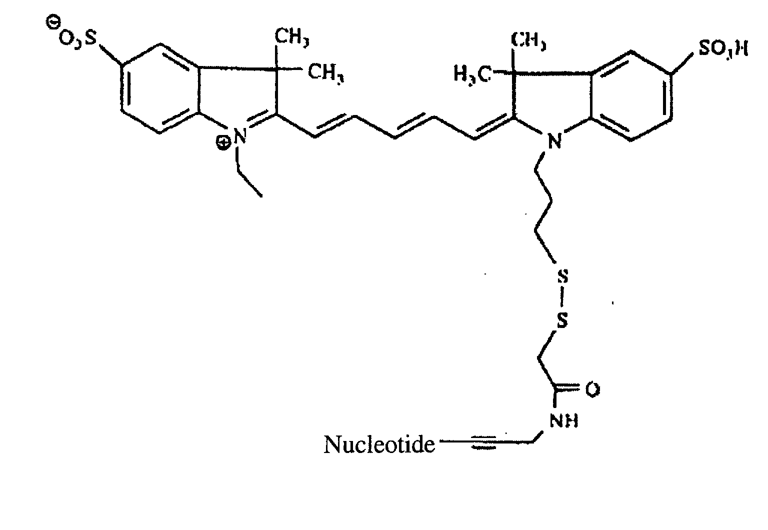 wherein Nucleotide is as defined in claim 1.

**5.** A nucleotide complex according to claim 4, **characterised in that** Nucleotide is chosen from the group of dATP, dGTP, dCTP and dTTP.

**6.** A nucleotide complex according to claim 5, which is

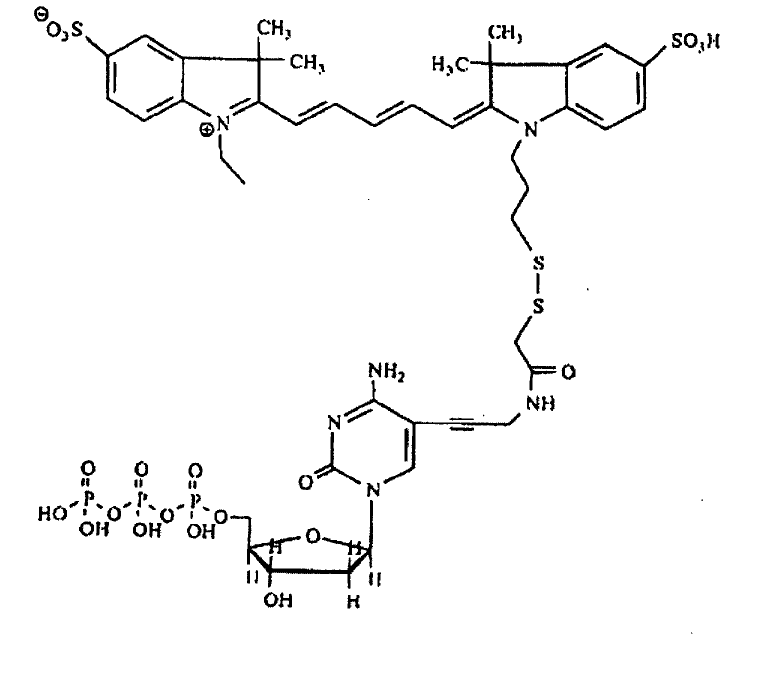

**7.** A method utilizing the dye labeled nucleotide complex of claim 1 to determining the nucleotide sequence of the features of an array of immobilized nucleic acids comprising the steps of:
a) adding a mixture comprising an oligonucleotide primer and a template-dependent polymerase to an array of immobilized nucleic acid features under conditions permitting hybridization of the primer to the immobilized nucleic acids;
b) adding a single, fluorescently labeled deoxynucleoside triphosphate with a cleavable linkage to the mixture under conditions which permit incorporation of the labeled deoxynucleotide onto the 3' end of the primer if it is complementary to the next adjacent base in the sequence to be determined;
c) detecting incorporated label by monitoring fluorescence;
d) repeating steps (b) - (c) with each of the remaining three labeled deoxynucleoside triphosphates with a cleavable linkage in turn; and
e) repeating steps (b) - (d) until the nucleotide sequence is determined.

**8.** The method of claim 7 wherein the primer, buffer and polymerase are cast into a polyacrylamide gel bearing the array of immobilized nucleic acids.

**9.** The method of claim 7 wherein single-stranded binding protein is present during step (b).

**10.** The method of claim 7 wherein said single fluorescently labeled deoxynucleotide with a cleavable linkage further comprises a mixture of the single deoxynucleoside triphosphate in labeled and unlabeled forms.

**11.** The method of claim 7 wherein after step (d) and before step (e) the additional step of photobleaching said array is performed.

**12.** The method of claim 7 wherein after step (d) and before step (e) the additional step of cleaving said linkage to the fluorophore is performed.

**13.** The method of claim 7 wherein after step (c) and before step (d) the additional step of cleaving said linkage to the fluorophore is performed.

**14.** The method of claim 12 or 13 wherein said step of cleaving comprises contacting said linkage with a reducing agent.

**15.** The method of claim 14 wherein said reducing agent is dithiothreitol.
